# EUROPÄISCHE PATENTSCHRIFT

(11) **EP 0 847 996 B1**
(45) Veröffentlichungstag und Bekanntmachung des Hinweises auf die Patenterteilung: **19.03.2003**
(21) Anmeldenummer: 97121437.4
(22) Anmeldetag: 05.12.1997
(51) Int. Cl.: C07D 239/94, A61K 31/33, C07D 217/22, C07D 215/42, C07D 265/16

(54) **Sulfonamid-substituierte Verbindungen als K-Kanal-Blocker**
Sulfonamide substituted compounds as K-channel blockers
Composés portant un groupe sulfonamide comme agents bloquant des canaux K

(30) Priorität: 16.12.1996 DE 19652213; 15.07.1997 DE 19730326
(43) Veröffentlichungstag der Anmeldung: 17.06.1998
(73) Patentinhaber: HOECHST AKTIENGESELLSCHAFT, 65929 Frankfurt am Main (DE)
(72) Erfinder: Brendel, Joachim, Dr., 61118 Bad Vilbel (DE); Lang, Hans Jochen, Dr., 65719 Hofheim (DE); Gerlach, Uwe, Dr., 65795 Hattersheim (DE); Weidmann, Klaus, Dr., 61476 Kronberg (DE)

(56) Entgegenhaltungen:
- EP-A- 0 389 861
- EP-A- 0 655 448
- EP-A- 0 807 629
- LOHRMANN E ET AL: "A NEW CLASS OF INHIBITORS OF CAMP-MEDIATED CL- SECRETION IN RABBIT COLON, ACTING BY THE REDUCTION OF CAMP-ACTIVATED K+ CONDUCTANCE" PFLUEGERS ARCHIV, Bd. 429, 1995, Seiten 517-530, XP002038768

## Beschreibung

Die Erfindung betrifft Verbindungen der Formel I, 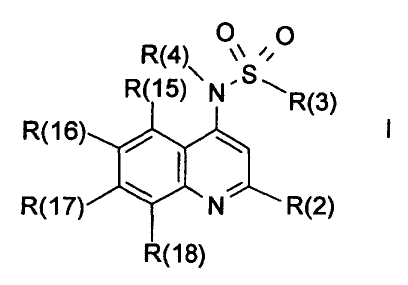 worin R(2), R(3), R(4), R(15), R(16), R(17) und R(18) die im folgenden angegebenen Bedeutungen haben, ihre Herstellung und ihre Verwendung, insbesondere in Arzneimitteln. Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acylaminochroman-Derivate intensiv bearbeitet, wobei auch einige entsprechende Chromenderivate beschrieben wurden. Prominentester Vertreter dieser Klasse ist das Cromakalim der Formel A, ein Beispiel für ein Chromen ist die Verbindung der Formel B (J. Chem. Soc. Perkin Trans.1, 1991, 63-70).

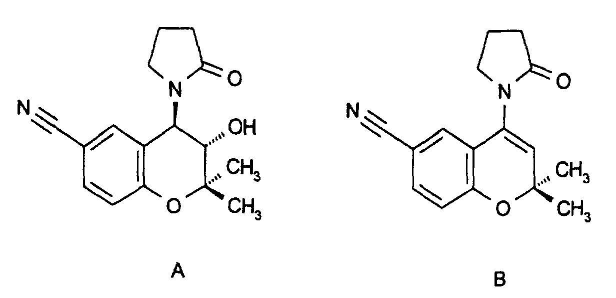

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺lonen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Von diesen Acylamino-Derivaten unterscheiden sich die erfindungsgemäßen Verbindungen der Formel I strukturell unter anderem durch den Ersatz der Acylaminogruppe durch eine Sulfonylaminofunktion. Während das Cromakalim (Formel A) sowie das Chromenderivat der Formel B und analoge Acylamino-Verbindungen als Öffner ATP-sensitiver K⁺-Kanäle wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺(cAMP) -Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit.

Neben den oben genannten Cromakalim- bzw. Acylaminochroman-Derivaten werden in der Literatur auch Verbindungen mit 4-Sulfonylaminochroman-Struktur beschrieben, die sich aber sowohl in der Struktur als auch in der biologischen Wirkung deutlich von den erfindungsgemäßen Verbindungen der Formel I unterscheiden. So werden in der EP-A-315 009 Chromanderivate mit 4-Phenylsulfonylaminostruktur beschrieben, die sich durch antithrombotische und antiallergische Eigenschaften auszeichnen. In der EP-A-389 861 und der JP 01294677 werden 3-Hydroxychroman- bzw. Chromen-Derivate mit einer cyclischen 4-Sulfonylaminogruppe beschrieben (z.B. Verbindung C), die über eine Aktivierung des K⁺(ATP)-Kanals als Antihypertensiva wirken sollen. In der EP-A-370 901 werden 3-Hydroxychroman- bzw. Chromen-Derivate mit einer 4-Sulfonylaminogruppe, wobei die restliche Valenz des N-Atoms ein Wasserstoffatom trägt, beschrieben, die über ZNS-Wirkungen verfügen. Weitere 4-Sulfonylaminochroman-Derivate werden in Bioorg. Med. Chem. Lett. 4 (1994), 769 - 773: "N-sulfonamides of benzopyranrelated potassium channel openers: conversion of glyburyde insensitive smooth muscle relaxants to potent smooth muscle contractors" beschrieben.

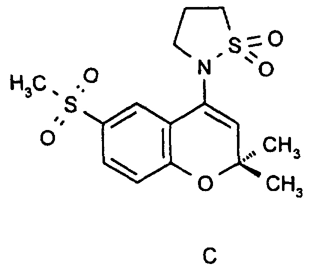

Die vorliegende Erfindung betrifft Verbindungen der Formel I, 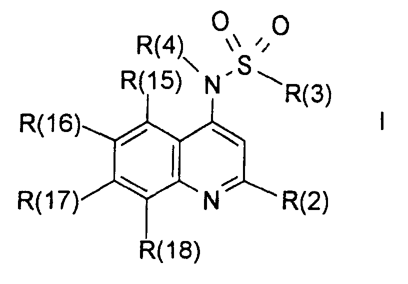 worin bedeuten:
- R(2): Wasserstoff, CF₃, F, Cl, Methoxy, Alkyl mit 1, 2 oder 3 C-Atomen;
- R(3): R(12)-CₙH₂ₙ-;
R(12) Methyl;
n Null, 1 oder 2;
- R(4): R(14)-CᵣH₂ᵣ,
wobei eine CH₂-Gruppe der Gruppe CᵣH₂ᵣ ersetzt sein kann durch -O-, -CO-, -CO-O-, -O-CO-, -NR(10b)- oder -CONR(10b)-;
R(10b) Wasserstoff oder Alkyl mit 1, 2 oder 3 C-Atomen;
R(14) Methyl, CF₃, -NR(23)R(24), 1-Piperidyl, 1-Pyrrolidinyl, 4-Morpholinyl, 4-Methylpiperazin-1-yl, Pyridyl, Thienyl oder Imidazolyl,
wobei Pyridyl, Thienyl und Imidazolyl unsubstituiert sind oder substituiert mit 1 oder 2 Substituenten, ausgewählt aus der Gruppe bestehend aus F, Cl, CF₃, OH, Methyl, Methoxy, Dimethylamino, Sulfamoyl und Methylsulfonyl;
R(23) und R(24) unabhängig voneinander Wasserstoff oder Alkyl mit 1, 2 oder 3 C-Atomen;
r Null, 1, 2, 3, 4, 5, 6, 7 oder 8;
- R(15), R(16), R(17) und R(18): unabhängig voneinander Wasserstoff, F, Cl, Br, I, Alkyl mit 1, 2 oder 3 C-Atomen, -CN, -CF₃, -NO₂, -Z-CₛH₂ₛ-R(22), Thienyl oder Phenyl,
das unsubstituiert ist oder substituiert mit 1 oder 2 Substituenten, ausgewählt aus der Gruppe bestehend aus F, Cl, CF₃, OH, Methyl, Methoxy, Sulfamoyl und Methylsulfonyl;
Z -O-, -CO-, -CO-O-, -O-CO-, -SO₂-, -SO₂-O-, -SO₂NR(10c),
-NR(10c)- oder -CONR(10c)-;
R(10c) Wasserstoff oder Methyl;
s Null, 1, 2, 3 oder 4;
R(22) Wasserstoff, CF₃, Cycloalkyl mit 5 oder 6 C-Atomen, -NR(19)R(20) oder Phenyl,
das unsubstituiert ist oder substituiert mit 1 oder 2 Substituenten, ausgewählt aus der Gruppe bestehend aus F, Cl, CF₃, OH, Methyl, Methoxy, Dimethylamino, Sulfamoyl und Methylsulfonyl;
R(19) und R(20) unabhängig voneinander Wasserstoff oder Alkyl mit 1 oder 2 C-Atomen,
sowie ihre physiologisch verträglichen Salze.

Alkylreste und Alkylenreste können geradkettig oder verzweigt sein. Dies gilt auch für die Alkylenreste der Formeln CₙH₂ₙ, CᵣH₂ᵣ und CₛH₂ₛ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Monosubstituierte Phenylreste können in der 2-, der 3- oder der 4-Position substituiert sein, disubstituierte in der 2,3-, 2,4-, 2,5-, 2,6-, 3,4- oder 3,5-Position. Entsprechendes gilt für Thienyl-, Pyridyl- und Imidazolylreste. Thienylreste können in der 2- oder der 3-Position, Pyridylreste in der 2-, 3- oder 4-Position, Imidazolylreste in der 1-, 2-, 4- oder 5-Position gebunden sein. Bei Disubstitution eines Restes können die Substituenten gleich oder verschieden sein.

Enthalten die Verbindungen der Formel I eine oder mehrere saure oder basische Gruppen bzw. einen oder mehrere basische Heterocyclen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Racematen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar, die ebenfalls Gegenstand der vorliegenden Erfindung sind. So erhält man beispielsweise eine Verbindung der Formel I, indem man
a) eine Verbindung der Formel II, 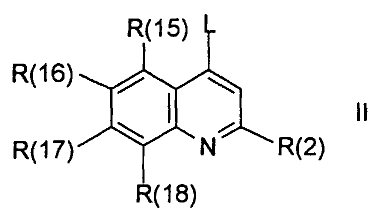 worin R(2), R(15), R(16), R(17) und R(18) die oben angegebenen Bedeutungen besitzen und L eine nucleofuge Fluchtgruppe, insbesondere F, Cl, Br, I, Methansulfonyloxy oder p-Toluolsulfonyloxy, bedeutet, in an sich bekannter Weise umsetzt mit einem Sulfonamid oder dessen Salz der Formel III, 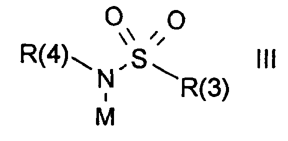 worin R(3) und R(4) die oben angegebenen Bedeutungen besitzen und M für Wasserstoff oder vorzugsweise für ein Metalläquivalent, besonders bevorzugt für Lithium, Natrium oder Kalium steht; oder daß man
b) eine Verbindung der Formel IV 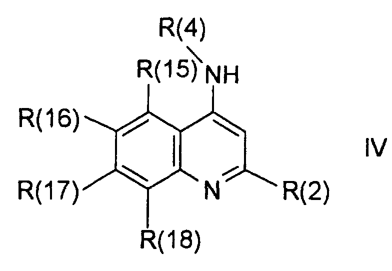 worin R(2), R(4), R(11), R(15), R(16), R(17) und R(18) die oben angegebenen Bedeutungen besitzen, mit einem Sulfonsäure-Derivat der Formel V 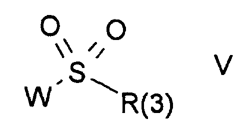 umsetzt, worin R(3) die oben angegebenen Bedeutungen besitzt und W eine nucleofuge Fluchtgruppe, wie z. B. Fluor, Brom, 1-Imidazolyl, insbesondere aber Chlor, bedeutet;
   oder daß man
c) eine Verbindung der Formel VI 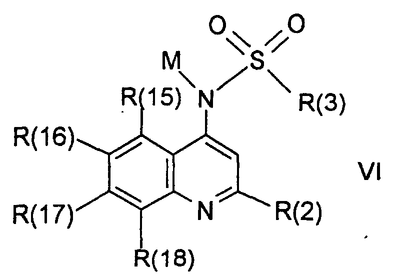 worin R(2), R(3), R(15), R(16), R(17), R(18) und M die oben angegebenen Bedeutungen besitzen, in an sich bekannter Weise im Sinne einer Alkylierungsreaktion mit einem Alkylierungsmittel der Formel VII umsetzt,

   R(4)-L VII

   worin R(4) die oben angegebenen Bedeutungen mit Ausnahme von Wasserstoff besitzt und L die oben angegebenen Bedeutungen besitzt;
   oder daß man
d) in einer Verbindung der Formel I 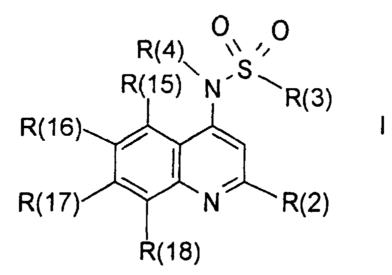
worin R(2), R(3), R(4), R(15), R(16), R(17) und R(18) die angegebenen Bedeutungen besitzen, in mindestens einer der Positionen R(15), R(16), R(17), R(18) des Ringsystems R(5)-R(6) eine elektrophile Substitutionsreaktion durchführt, sofern diese Position Wasserstoff bedeutet.

Die Verfahrensweise a) entspricht der nucleophilen Substitution einer Abgangsgruppe in einem reaktiven Bicyclus der Formel II durch ein Sulfonamid bzw. eines seiner Salze der Formel III. Wegen der höheren Nucleophilie und höheren Reaktivität eines in der Salzform vorliegenden Sulfonamids ist bei Verwendung eines freien Sulfonamids (Formel III, M = H) bevorzugt, aus diesem zunächst durch Einwirkung einer Base ein Sulfonamidsalz (Formel III, M = Metallkation) zu erzeugen. Setzt man ein freies Sulfonamid (Formel III, M = H) ein, so kann die Deprotonierung des Sulfonamids zum Salz in situ erfolgen. Bevorzugt werden solche Basen verwandt, die selbst nicht oder nur wenig alkyliert werden, wie z. B. Natriumcarbonat, Kaliumcarbonat, sterisch stark gehinderte Amine, z. B. Dicyclohexylamin, N,N-Dicyclohexyl-ethylamin, oder andere starke Stickstoffbasen mit geringer Nucleophilie, beispielsweise DBU (Diazabicycloundecen), N,N',N"'-Triisopropylguanidin etc. Es können allerdings auch andere üblich verwendete Basen für die Reaktion eingesetzt werden, wie Kalium-tert-butylat, Natriummethylat, Alkalihydrogencarbonate, Alkalihydroxide, wie beispielsweise LiOH, NaOH oder KOH, oder Erdalkalihydroxide, wie beispielsweise Ca(OH)₂.

Vorzugsweise arbeitet man in einem Lösungsmittel, besonders bevorzugt in polaren organischen Lösungsmitteln wie z. B. Dimethylformamid (DMF), Dimethylacetamid (DMA), Dimethylsulfoxid (DMSO), Tetramethylharnstoff (TMU), Hexamethylphosphorsäuretriamid (HMPT), Tetrahydrofuran (THF), Dimethoxyethan (DME) oder anderen Ethern, oder beispielsweise auch in einem Kohlenwasserstoff wie Toluol oder in einem halogenierten Kohlenwasserstoff wie Chloroform oder Methylenchlorid usw. Es kann aber auch in polaren protischen Lösungsmitteln gearbeitet werden, wie z. B. in Wasser, Methanol, Ethanol, Isopropanol, Ethylenglykol oder dessen Oligomeren und deren entsprechenden Halbethern oder auch deren Ethern. Die Reaktion kann auch in Gemischen dieser Lösungsmittel durchgeführt werden. Ebenso kann die Reaktion aber auch ganz ohne Lösungsmittel durchgeführt werden. Die Reaktion wird bevorzugt in einem Temperaturbereich von -10 bis +140 °C, besonders bevorzugt im Bereich von 20 bis 100 °C durchgeführt. In günstiger Weise kann Verfahrensweise a) auch unter den Bedingungen einer Phasentransferkatalyse durchgeführt werden.

Die Verbindungen der Formel II gewinnt man nach literaturbekannten Methoden, beispielsweise aus den entsprechenden 4-Oxoverbindungen der Formel X, 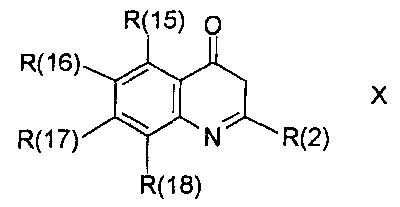 worin R(2), R(15), R(16), R(17) und R(18) die oben angegebenen Bedeutungen besitzen, bzw. deren tautomerer Form (Formel II, L = -OH) durch Einwirkung eines anorganischen Säurehalogenids, wie beispielsweise POCl₃, PCl₃, PCl₅, SOCl₂, SOBr₂, COCl₂ oder Gemischen derselben. Vielfach hat sich die Verwendung katalytisch wirkender Zusätze, wie DMF bei Verwendung von SOCl₂ oder N,N-Dimethylanilin bei Verwendung von POCl₃ bewährt. Vorteilhaft arbeitet man in einem Lösungsmittel, das ausreichend inert gegenüber diesen halogenierenden energiereichen Reagenzien ist, wie beispielsweise in Toluol oder einem halogenierten Kohlenwasserstoff wie z.B. in Chloroform, Methylenchlorid oder in einem der flüssigen Halogenierungsmittel selbst, bevorzugt in POCl₃.

Verfahrensweise b)
beschreibt die an sich bekannte und häufig angewandte Reaktion einer reaktiven Sulfonylverbindung der Formel V, insbesondere einer Chlorsulfonylverbindung (W = Cl), mit einem Amino-Derivat der Formel IV zum entsprechenden Sulfonamid-Derivat der Formel I. Die Reaktion kann prinzipiell ohne Lösungsmittel durchgeführt werden, jedoch werden derartige Reaktionen in den meisten Fällen unter Verwendung eines Lösungsmittels durchgeführt.

Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.

Die Amino-Derivate der Formel IV erhält man in an sich literaturbekannter Weise bevorzugt durch Reaktion der reaktiven Verbindungen der Formel II mit R(1), R(2), R(5), R(6), X, Y und L in der angegebenen Bedeutung, entweder mit Ammoniak oder einem Amin der Formel XI

R(4)-NH₂ XI

mit R(4) in der angegebenen Bedeutung.

Verfahrensweise c)
repräsentiert die an sich bekannte Alkylierungsreaktion eines Sulfonamids bzw. eines seiner Salze VI mit einem Alkylierungsmittel der Formel VII. Entsprechend der Reaktionsanalogie mit Verfahrensweise a) gelten für Verfahrensweise c) die bereits ausführlich unter Verfahrensweise a) beschriebenen Reaktionsbedingungen.

Die Herstellung der Sulfonamid-Derivate VI und deren Vorprodukte wurden bereits bei Verfahrensweise b) beschrieben. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

Verfahrensweise d)
beschreibt die weitere chemische Umwandlung von erfindungsgemäßen Verbindungen der Formel I in andere Verbindungen der Formel I durch elektrophile Substitutionsreaktionen in einer oder in mehreren der mit R(15) bis R(18) bezeichneten Positionen des Ringsystems R(5)-R(6), die jeweils Wasserstoff bedeuten.

Bevorzugte Substitutionsreaktionen sind
1. die aromatische Nitrierung zur Einführung einer oder mehrerer Nitrogruppen, die in nachfolgenden Reaktionen teilweise oder alle zu Aminogruppen reduziert werden können. Die Aminogruppen können wiederum in nachfolgenden Reaktionen in andere Gruppen umgewandelt werden, beispielsweise in einer Sandmeyerreaktion, z. B. zur Einführung von Cyanogruppen;
2. die aromatische Halogenierung insbesondere zur Einführung von Chlor, Brom oder Jod;
3. die Chlorsulfonierung, z.B. durch Einwirkung von Chlorsulfonsäure, zur Einführung einer Chlorsulfonylgruppe, die in nachfolgenden Reaktionen in andere Gruppen umgewandelt werden kann, z. B. in eine Sulfonamidgruppe;
4. die Friedel-Crafts-Acylierungsreaktion zur Einführung eines Acylrestes oder eines Sulfonylrestes durch Einwirkung der entsprechenden Säurechloride in Gegenwart einer Lewis-Säure als Friedel-Crafts-Katalysator, vorzugsweise in Gegenwart von wasserfreiem Aluminiumchlorid.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Es wurde bereits gesagt, daß die Verbindungen der Formel I überraschenderweise eine starke und spezifische blockierende (schließende) Wirkung auf einen K⁺-Kanal haben, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine antidiarrhoische Wirkung aus und sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien, vor allem von Herzrhythmusstörungen, die durch Aktionspotential-Verlängerung behoben werden können. Sie können speziell angewandt werden zur Therapie und Prophylaxe von atrialer Fibrillation (Vorhoff-Flimmern) und atrialem Flattern (Vorhoff-Flattern) sowie zur Therapie und Prophylaxe von Reentry-Arrhythmien und zur Verhinderung des plötzlichen Herztodes infolge von Kammerflimmern.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht. Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III-Antiarrhythmika blockieren überwiegend oder ausschließlich I_{Kr} (z.B. Dofetilid, d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44B-49B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T.J. Colatsky, C.H. Follmer und C.F. Starmer; "Channel Specificity in Antiarrhythmic Drug Action; Mechanism of potassium channel block and its role in suppressing and aggravating cardiac arrhythmias"; Circulation 82 (1990), 2235 - 2242; A.E. Busch, K. Malloy, W.J. Groh, M.D. Varnum, J.P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys. Res. Commun. 202 (1994), 265 - 270).

Darüber hinaus tragen die Verbindungen zu einer deutlichen Verbesserung der Herzinsuffizienz, insbesondere der Stauungsherzinsuffizienz (Congestive Heart Failure) bei, vorteilhafterweise in der Kombination mit kontraktionsfördernden (positiv inotropen) Wirkstoffen, z.B. Phosphordiesterasehemmern.

Trotz der therapeutisch nutzbaren Vorteile, die durch eine Blockade des I_{Ks} erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1:10). In der WO-A-95/14470 wird die Verwendung von Benzodiazepinen als selektive Blocker des I_{Ks} beansprucht. Weitere I_{Ks}-Blocker sind beschrieben in FEBS Letters 396 (1996), 271-275: "Specific blockade of slowly activating I_{sK} channels by chromanols ..." und Pflügers Arch. - Eur. J. Physiol. 429 (1995), 517-530: "A new class of inhibitors of cAMPmediated Cl- secretion in rabbit colon, acting by the reduction of cAMP-activated K⁺ conductance". Die Potenz der dort genannten 3-Hydroxychromanole ist jedoch geringer als die der erfindungsgemäßen Verbindungen der Formel I. Darüber hinaus haben die erfindungsgemäßen Verbindungen der Formel I gegenüber den 3-Hydroxychromanolen den Vorteil, daß die Sulfonamidfunktion nicht an einem chiralen C-Atom steht, so daß im Gegensatz zu den bekannten Verbindungen keine aufwendige enantioselektive Synthese oder Racematspaltung zur Produktion eines einheitlichen Wirkstoffs erforderlich ist.

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Exchange-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H₂-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

Die Verbindungen der Formel I und ihre physiologisch verträglichen Salze hemmen selektiv K⁺(cAMP)-Kanäle bzw. I_{Ks}-Kanäle. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine Beeinflussung von Kaliumkanälen beabsichtigt ist, sowie für diagnostische Zwecke, z. B. in der in vitro-Diagnostik von Zell- oder Gewebsproben. Ferner können sie, wie bereits oben erwähnt, als Zwischenprodukte zur Herstellung weiterer Arzneimittelwirkstoffe eingesetzt werden.

### Experimenteller Teil

Liste der Abkürzungen
- DMA: N,N-Dimethylacetamid
- F.p.: Schmelzpunkt
- RT: Raumtemperatur

### Beispiel 1: 7-Chlor-4-(N-ethylsulfonyl-N-methyl)amino-chinolin

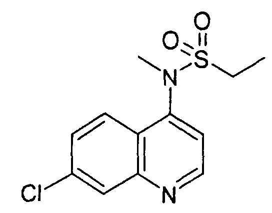

Zu einer Aufschlämmung von 0,22 g (7,6 mmol) Natriumhydrid (als 80%ige Dispersion in Öl) in 10 ml wasserfreiem DMA wurde unter Argon-Schutzgasatmosphäre eine Lösung von 0,62 g (5 mmol) Ethylsulfonsäure-N-methylamid in 10 ml wasserfreiem DMA zugetropft. Man ließ 1 Stunde bei Raumtemperatur rühren, fügte anschließend 1,09 g (5,5 mmol) 4,7-Dichlorchinolin zu und erhitzte 6 h auf 120°C. Das Lösungsmittel wurde unter vermindertem Druck abdestilliert, der Rückstand mit Wasser verrührt und der ausgefallene Feststoff abgesaugt. Nach Reinigung des Rohproduktes durch Chromatographie an Kieselgel wurden 0,08 g 7-Chlor-4-(N-ethylsulfonyl-N-methyl)amino-chinolin erhalten; F.p. 273°C.

### Beispiel 2: 4-(N-Ethylsulfonyl-N-methyl)amino-2-trifluormethyl-chinolin

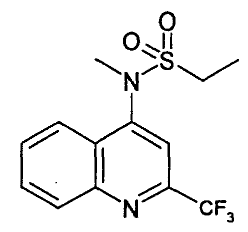

Zu einer Aufschlämmung von 0,165 g (5,5 mmol) Natriumhydrid (als 80%ige Dispersion in Öl) in 10 ml wasserfreiem DMA wurde unter Argon-Schutzgasatmosphäre eine Lösung von 0,67 g (5,5 mmol) Ethylsulfonsäure-N-methylamid in 10 ml wasserfreiem DMA zugetropft. Man ließ 4 Stunden bei RT rühren, fügte anschließend eine Lösung von 1,15 g (5,0 mmol) 4-Chlor-2-trifluormethyl-chinolin zu und rührte noch 3 Tage bei RT. Das Lösungsmittel wurde unter vermindertem Druck abdestilliert, der Rückstand mit Wasser versetzt und mit Essigester extrahiert. Nach Reinigung des Rohproduktes durch Chromatographie an Kieselgel mit Essigester/Cyclohexan 1 : 1 wurden 0,6 g 4-(N-Ethylsulfonyl-N-methyl)amino-2-trifluormethyl-chinolin als gelbliches Öl erhalten.

### Beispiel 3: 8-Chlor-4-(N-ethylsulfonyl-N-methyl)amino-2-trifluormethyl-chinolin

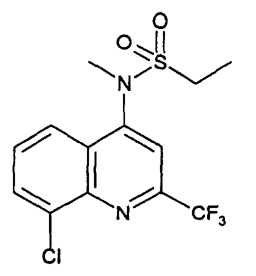

Analog Beispiel 5 wurden aus 1,33 g 4,8-Dichlor-2-trifluormethyl-chinolin 0,5 g 8-Chlor-4-(N-ethylsulfonyl-N-methyl)amino-2-trifluormethyl-chinolin als Öl erhalten.

## Patentansprüche

1. Verbindungen der Formel I, 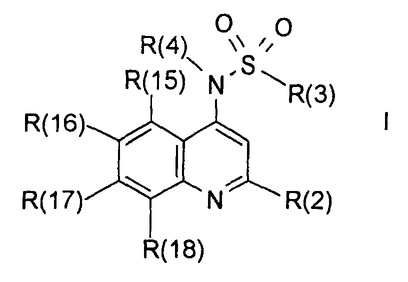 worin bedeuten:
R(2) Wasserstoff, CF₃, F, Cl, Methoxy, Alkyl mit 1, 2 oder 3 C-Atomen;
R(3) R(12)-CₙH₂ₙ-;
R(12) Methyl;
n Null, 1 oder 2;
R(4) R(14)-CᵣH₂ᵣ,
wobei eine CH₂-Gruppe der Gruppe CᵣH₂ᵣ ersetzt sein kann durch -O-, -CO-, -CO-O-, -O-CO-, -NR(10b)- oder -CONR(10b)-;
R(10b) Wasserstoff oder Alkyl mit 1, 2 oder 3 C-Atomen;
R(14) Methyl, CF₃, -NR(23)R(24), 1-Piperidyl, 1-Pyrrolidinyl, 4-Morpholinyl, 4-Methylpiperazin-1-yl, Pyridyl, Thienyl oder Imidazolyl,
wobei Pyridyl, Thienyl und Imidazolyl unsubstituiert sind oder substituiert mit 1 oder 2 Substituenten, ausgewählt aus der Gruppe bestehend aus F, Cl, CF₃, OH, Methyl, Methoxy, Dimethylamino, Sulfamoyl und Methylsulfonyl;
R(23) und R(24) unabhängig voneinander Wasserstoff oder Alkyl mit 1, 2 oder 3 C-Atomen;
r Null, 1, 2, 3, 4, 5, 6, 7 oder 8;
R(15), R(16), R(17) und R(18) unabhängig voneinander Wasserstoff, F, Cl, Br, I, Alkyl mit 1, 2 oder 3 C-Atomen, -CN, -CF₃, -NO₂, -Z-CₛH₂ₛ-R(22), Thienyl oder Phenyl,
das unsubstituiert ist oder substituiert mit 1 oder 2 Substituenten, ausgewählt aus der Gruppe bestehend aus F, Cl, CF₃, OH, Methyl, Methoxy, Sulfamoyl und Methylsulfonyl;
Z -O-, -CO-, -CO-O-, -O-CO-, -SO₂-, -SO₂-O-, -SO₂NR(10c),
-NR(10c)- oder -CONR(10c)-;
R(10c) Wasserstoff oder Methyl;
s Null, 1, 2, 3 oder 4;
R(22) Wasserstoff, CF₃, Cycloalkyl mit 5 oder 6 C-Atomen, -NR(19)R(20) oderPhenyl,
das unsubstituiert ist oder substituiert mit 1 oder 2 Substituenten, ausgewählt aus der Gruppe bestehend aus F, Cl, CF₃, OH, Methyl, Methoxy, Dimethylamino, Sulfamoyl und Methylsulfonyl;
R(19) und R(20) unabhängig voneinander Wasserstoff oder Alkyl mit 1 oder 2 C-Atomen,
sowie ihre physiologisch verträglichen Salze.

2. Verfahren zur Herstellung von Verbindungen der Formel I gemäß Anspruch 1, **dadurch gekennzeichnet, daß** man
a) eine Verbindung der Formel II, 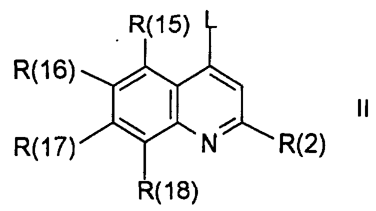 worin R(2), R(15), R(16), R(17) und R(18) die in den Anspruch 1 angegebenen Bedeutungen besitzen und L eine nucleofuge Fluchtgruppe bedeutet, umsetzt mit einem Sulfonamid oder dessen Salz der Formel III, 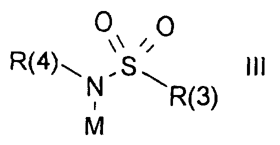 worin R(3) und R(4) die in Anspruch 1 angegebenen Bedeutungen besitzen und M für Wasserstoff oder vorzugsweise für ein Metalläquivalent steht;
oder daß man
b) eine Verbindung der Formel IV 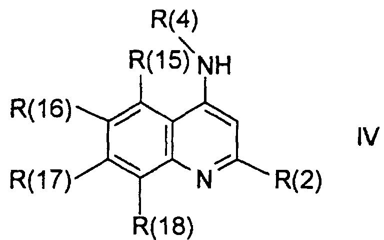 worin R(2), R(2), R(11), R(15), R(16), R(17) und R(18) die in den Anspruch 1 angegebenen Bedeutungen besitzen, mit einem Sulfonsäure-Derivat der Formel V 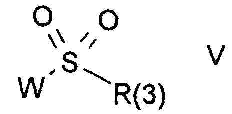 umsetzt, worin R(3) die in Anspruch 1 angegebenen Bedeutungen besitzt und W eine nucleofuge Fluchtgruppe bedeutet;
oder daß man
c) eine Verbindung der Formel VI 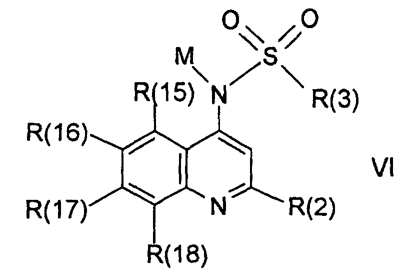 worin R(2), R(3), R(15), R(16), R(17), R(18) und M die in Anspruch 1 bzw. unter a) angegebenen Bedeutungen besitzen, mit einem Alkylierungsmittel der Formel VII umsetzt,
R(4)-L VII
worin R(4) die in Anspruch 1 angegebenen Bedeutungen mit Ausnahme von Wasserstoff besitzt und L die unter a) angegebenen Bedeutungen besitzt;
oder daß man
d) in einer Verbindung der Formel I 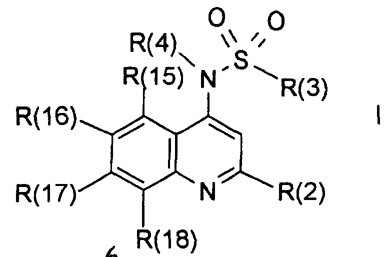
worin R(2), R(3), R(4), R(15), R(16), R(17) und R(18) die in Anspruch 1 angegebenen Bedeutungen besitzen, in mindestens einer der Positionen R(15), R(16), R(17), R(18) eine elektrophile Substitutionsreaktion durchführt, sofern diese Position Wasserstoff bedeutet.

3. Verbindungen der Formel I gemäß Anspruch 1 und ihre physiologisch verträglichen Salze zur Anwendung als Arzneimittel.

4. Pharmazeutische Zubereitung, enthaltend eine wirksame Menge mindestens einer Verbindung der Formel I nach Anspruch 1 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

5. Verwendung einer Verbindung der Formel I nach Anspruch 1 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

6. Verwendung einer Verbindung der Formel I nach Anspruch 1 und/oder eines physiologisch verträglichen Salzes davon zur Herstellung eines Medikaments zum Inhibieren der Magensäuresekretion.

7. Verwendung einer Verbindung der Formel I nach Anspruch 1 und/oder eines physiologisch verträglichen Salzes davon zur Herstellung eines Medikaments zur Therapie oder Prophylaxe von Ulcera des Magens oder des intestinalen Bereiches.

8. Verwendung einer Verbindung der Formel I nach Anspruch 1 und/oder eines physiologisch verträglichen Salzes davon zur Herstellung eines Medikaments zur Therapie oder Prophylaxe der Refluxösöphagitis.

9. Verwendung einer Verbindung der Formel I nach Anspruch 1 und/oder eines physiologisch verträglichen Salzes davon zur Herstellung eines Medikaments zur Therapie oder Prophylaxe von Durchfallerkrankungen.

10. Verwendung einer Verbindung der Formel I nach Anspruch 1 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

11. Verwendung einer Verbindung der Formel I nach Anspruch 1 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

12. Verwendung einer Verbindung der Formel I nach Anspruch 1 und/oder eines physiologisch verträglichen Salzes davon zur Herstellung eines Medikaments zur Therapie oder Prophylaxe von atrialer Fibrillation oder atrialem Flattern.

13. Verwendung einer Verbindung der Formel I nach Anspruch 1 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmern.

14. Verwendung einer Verbindung der Formel I nach Anspruch 1 und/oder eines physiologisch verträglichen Salzes davon zur Herstellung eines Medikaments zur Therapie der Herzinsuffizienz, insbesondere der Stauungsherzinsuffizienz (Congestive Heart Failure).

15. Verwendung einer Verbindung der Formel I nach Anspruch 1 und/oder eines physiologisch verträglichen Salzes davon zum Blockieren des Kaliumkanals, der durch cyclisches Adenosinmonophosphat (CAMP) geöffnet wird.

16. Verwendung einer Verbindung der Formel I nach Anspruch 1 und/oder eines physiologisch verträglichen Salzes davon zum Herstellen eines Medikaments zum Inhibieren der stimulierten Magensäuresekretion, zur Therapie oder Prophylaxe von Ulcera des Magens oder des intestinalen Bereiches, der Refluxösophagitis, von Durchfallerkrankungen, zur Therapie oder Prophylaxe von Arrhythmien, einschließlich atrialer, ventrikulärer und supraventrikulärer Arrhythmien, atrialer Fibrillation und atrialem Flattern und von Reentry-Arrhythmien, oder zur Verhinderung des plötzlichen Herztodes infolge von Kammerflimmern.

## Claims

1. A compound of the formula I 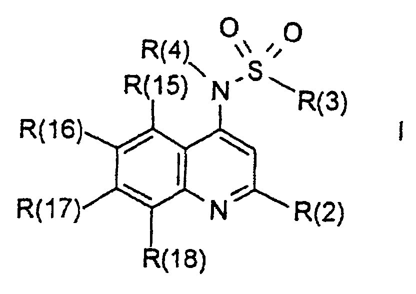 in which:
R(2) is hydrogen, CF₃, F, Cl, methoxy, alkyl having 1, 2 or 3 carbon atoms;
R(3) is R(12)-CₙH₂ₙ-;
R(12) is methyl;
n is zero, 1 or 2;
R(4) is R(14)-CᵣH₂ᵣ,
where a CH₂ group of the group CᵣH₂ᵣ can be replaced by -O-, -CO-, -CO-O -O-CO-, -NR(10b)- or -CONR(10b)-;
R(10b) is hydrogen or alkyl having 1, 2 or 3 carbon atoms;
R(14) is methyl, CF₃, -NR(23)R(24), 1-piperidyl, 1-pyrrolidinyl, 4-morpholinyl, 4-methylpiperazin-1-yl, pyridyl, thienyl or imidazolyl,
where pyridyl, thienyl and imidazolyl are unsubstituted or substituted by 1 or 2 substituents selected from the group consisting of F, Cl, CF₃, OH, methyl, methoxy, dimethylamino, sulfamoyl and methylsulfonyl;
R(23) and R(24) independently of one another are hydrogen or alkyl having 1, 2 or 3 carbon atoms;
r is zero, 1, 2, 3, 4, 5, 6, 7 or 8;
R(15), R(16), R(17) and R(18) independently of one another are hydrogen, F, Cl, Br, I, alkyl having 1, 2 or 3 carbon atoms, -CN, -CF₃, -NO₂, -Z-CₛH₂ₛ-R(22), thienyl or phenyl,
which is unsubstituted or substituted by 1 or 2 substituents selected from the group consisting of F, Cl, CF₃, OH, methyl, methoxy, sulfamoyl and methyl- sulfonyl;
Z is -O-, -CO-, -CO-O-, -O-CO-, -SO₂-, -SO₂-O-, -SO₂NR(10c), -NR(10c)- or -CONR(10c)-;
R(10c) is hydrogen or methyl;
s is zero, 1, 2, 3 or 4;
R(22) is hydrogen, CF₃, cycloalkyl having 5 or 6 carbon atoms, -NR(19)R(20) or phenyl,
which is unsubstituted or substituted by 1 or 2 substituents selected from the group consisting of F, Cl, CF₃, OH, methyl, methoxy, dimethylamino, sulfamoyl and methylsulfonyl;
R(19) and R(20) independently of one another are hydrogen or alkyl having 1 or 2 carbon atoms,
or its physiologically tolerable, salts.

2. A process for the preparation of compounds of the formula I as claimed in claim 1, which comprises
a) reacting a compound of the formula II 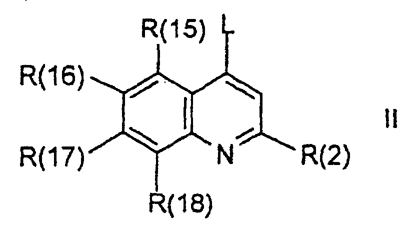 in which R(2), R(15), R(16), R(17) and R(18) have the meanings indicated in claim 1 and L is a nucleofugic leaving group, with a sulfonamide or its salt of the formula III 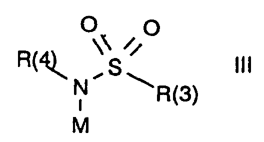 in which R(3) and R(4) have the meanings indicated in claim 1 and M is hydrogen or preferably a metal equivalent;
or by
b) reacting a compound of the formula IV 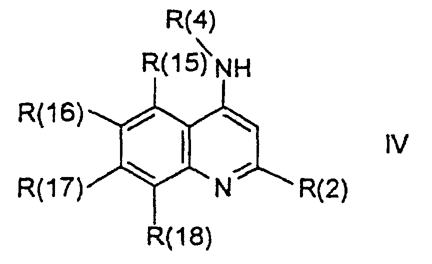 in which R(2), R(4), R(11), R(15), R(16), R(17) and R(18) have the meanings indicated in claim 1, with a sulfonic acid derivative of the formula V 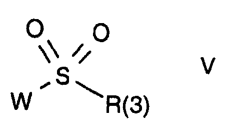 in which R(3) has the meanings indicated in claim 1 and W is a nucleofugic leaving group;
or by
c) reacting a compound of the formula VI 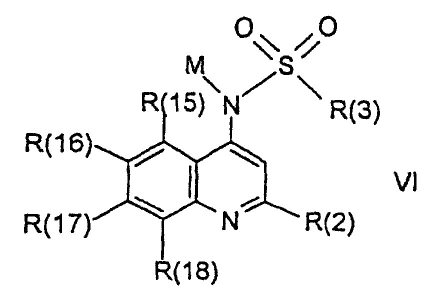 in which R(2), R(3), R(15), R(16), R(17), R(18) and M have the meanings indicated in claim 1 or under a), with an alkylating agent of the formula VII
R(4)-L VII
in which R(4) has the meanings indicated in claim 1 with the exception of hydrogen and L has the meanings indicated under a);
or by
d) carrying out, in a compound of the formula I 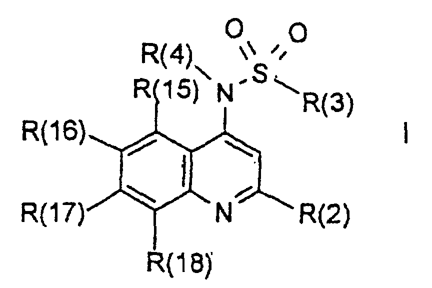 in which R(2), R(3), R(4), R(15), R(16), R(17) and R(18) have the meanings indicated in claim 1, an electrophilic substitution reaction in at least one of the positions R(15), R(16), R(17), R(18), if this position is hydrogen.

3. A compound of the formula I as claimed in claim 1 or its physiologically tolerable salts for use as a pharmaceutical.

4. A pharmaceutical preparation comprising an effective amount of at least one compound of the formula I as claimed in claim 1 and/or of a physiologically tolerable salt thereof as active compound, together with pharmaceutically acceptable excipients and additives and, if appropriate, additionally one or more other pharmacological active compounds.

5. The use of a compound of the formula I as claimed in claim 1 and/or of a physiologically tolerable salt thereof for the production of a medicament having K⁺ channel-blocking action for the therapy and prophylaxis of K⁺ channel-mediated diseases.

6. The use of a compound of the formula I as claimed in claim 1 and/or of a physiologically tolerable salt thereof for the production of a medicament for the inhibition of gastric acid secretion.

7. The use of a compound of the formula I as claimed in claim 1 and/or of a physiologically tolerable salt thereof for the production of a medicament for the therapy or prophylaxis of ulcers of the stomach or of the intestinal region.

8. The use of a compound of the formula I as claimed in claim 1 and/or of a physiologically tolerable salt 'thereof for the production of a medicament for the therapy or prophylaxis of reflux esophagitis.

9. The use of a compound of the formula I as claimed in claim 1 and/or of a physiologically tolerable salt thereof for the production of a medicament for the therapy or prophylaxis of diarrheal illnesses.

10. The use of a compound of the formula I as claimed in claim 1 and/or of a physiologically tolerable salt thereof for the production of a medicament for the therapy or prophylaxis of all types of arrhythmias, including atrial, ventricular and supraventricular arrhythmias.

11. The use of a compound of the formula I as claimed in claim 1 and/or of a physiologically tolerable salt thereof for the production of a medicament for the therapy or prophylaxis of cardiac arrhythmias which can be eliminated by action potential prolongation.

12. The use of a compound of the formula I as claimed in claim 1 and/or of a physiologically tolerable salt thereof for the production of a medicament for the therapy or prophylaxis of atrial fibrillation or atrial flutters.

13. The use of a compound of the formula I as claimed in claim 1 and/or of a physiologically tolerable salt thereof for the production of a medicament for the therapy or prophylaxis of reentry arrhythmias or for the prevention of sudden heart death as a result of ventricular fibrillation.

14. The use of a compound of the formula I as claimed in claim 1 and/or of a physiologically tolerable salt thereof for the production of a medicament for the therapy of cardiac insufficiency, in particular of congestive heart failure.

15. The use of a compound of the formula I as claimed in claim 1 and/or of a physiologically tolerable salt thereof for blocking the calcium channel which is opened by cyclic adenosine monophosphate (cAMP).

16. The use of a compound of the formula I as claimed in claim 1 and/or of a physiologically tolerable salt thereof for the production of a medicament for the inhibition of stimulated gastric acid secretion, for the therapy or prophylaxis of ulcers of the stomach or of the intestinal region, of reflux esophagitis, of diarrheal illnesses, for the therapy or prophylaxis of arrhythmias, including atrial, ventricular and supraventricular arrhythmias, atrial fibrillation and atrial flutters and of reentry arrhythmias, or for the prevention of sudden heart death as a result of ventricular fibrillation.

## Revendications

1. Composés de formule I 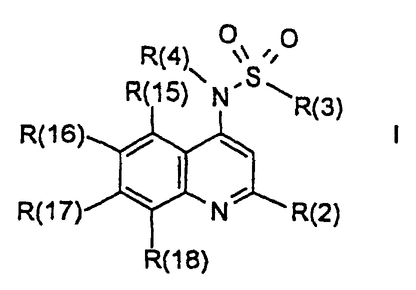 dans laquelle
R(2) représente un atome d'hydrogène ou un groupe CF₃, F, Cl, méthoxy ou alkyle de 1, 2 ou 3 atomes de carbone;
R(3) représente un groupe R(12)-CₙH₂ₙ,
R(12) est un groupe méthyle
n est égal à 0, 1 ou 2;
R(4) représente un groupe R(14)-CᵣH₂ᵣ, un groupe CH₂ du groupe CᵣH₂ᵣ pouvant être remplacé par -O-, -CO-, -CO-O-, -O-CO-, -NR(10b)- ou -CONR(10b)-;
R(10b) représente un atome d'hydrogène ou un groupe alkyle de 1, 2 ou 3 atomes de carbone;
R(14) représente un groupe méthyle, CF₃, -NR(23)R(24), 1-pipéridyle, 1-pyrrolidinyle, 4-morpholinyle, 4-méthylpipérazin-1-yle, pyridyle, thiényle ou imidazolyle, les groupes pyridyle, thiényle et imidazolyle étant non substitués ou substitués par 1 ou 2 substituants choisis dans le groupe constitué par F, Cl, CF₃, OH, méthyle, méthoxy, diméthylamino, sulfamoyle et méthylsulfonyle;
R(23) et R(24) représentent indépendamment l'un de l'autre un atome d'hydrogène ou un groupe alkyle de 1, 2 ou 3 atomes de carbone;
r est égal à 0, 1, 2, 3, 4, 5, 6, 7 ou 8;
R(15), R(16), R(17) et R(18) représentent, indépendamment les uns des autres, un atome d'hydrogène ou un groupe F, Cl, Br, I, alkyle de 1, 2 ou 3 atomes de carbone, -CN, -CF₃, -NO₂, -Z-CₛH₂ₛ-R(22), thiényle ou phényle, non substitué ou substitué par 1 ou 2 substituants choisis dans le groupe constitué par F, Cl, CF₃, OH, méthyle, méthoxy, sulfamoyle et méthylsulfonyle;
Z représente un groupe -O-, -CO-, -CO-O-, -O-CO-, -SO₂-, -SO₂-O-, -SO₂NR(10c), -NR(10c)- ou -CONR(10c)-;
R(10c) représente un atome d'hydrogène ou un groupe méthyle;
s est égal à 0,1,2, 3 ou 4;
R(22) représente un atome d'hydrogène ou un groupe CF₃, cycloalkyle de 5 ou 6 atomes de carbone, -NR(19)R(20) ou phényle qui est non substitué ou substitué par 1 ou 2 substituants choisis dans le groupe constitué par F, Cl, CF₃, OH, méthyle, méthoxy, diméthylamino, sulfamoyle et méthylsulfonyle;
R(19) et R(20) représentent indépendamment l'un de l'autre un atome d'hydrogène ou un groupe alkyle de 1 ou 2 atomes de carbone,
et leurs sels physiologiquement acceptables.

2. Procédé de préparation de composés de formule I selon la revendication 1, **caractérisé en ce que**
a) on fait réagir un composé de formule II 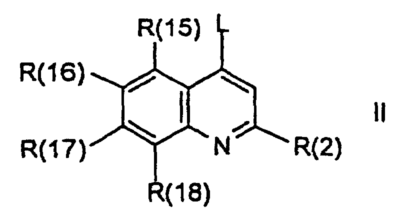 dans laquelle R(2), R(15), R(16), R(17) et R(18) ont la signification donnée dans la revendication 1 et L représente un groupe partant nucléofuge, avec un sulfonamide de formule III ou un de ses sels 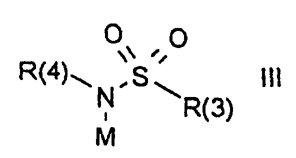 dans laquelle R(3) et R(4) ont la signification donnée dans la revendication 1 et M représente un atome d'hydrogène ou de préférence un équivalent de métal;
ou **en ce que**
b) on fait réagir un composé de formule IV 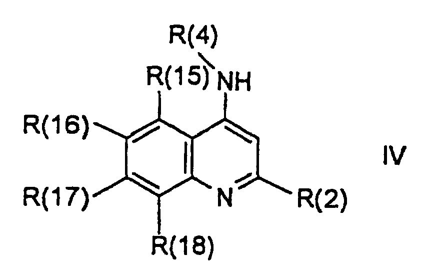 dans laquelle R(2), R(11), R(15), R(16), R(17) et R(18) ont la signification donnée dans la revendication 1, avec un dérivé d'acide sulfonique de formule V 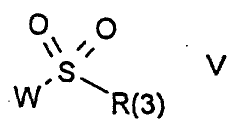 dans laquelle R(3) a la signification donnée dans la revendication 1 et W représente un groupe partant nucléofuge;
ou **en ce que**
c) on fait réagir un composé de formule VI 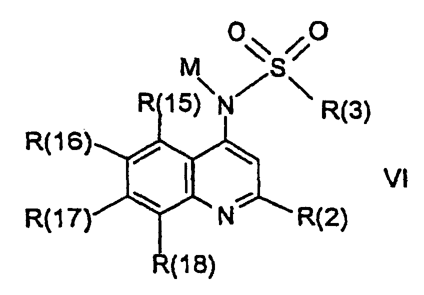 dans laquelle R(2), R(3), R(15), R(16), R(17), R(18) et M ont la signification donnée dans la revendication 1 et sous a), avec un agent d'alkylation de formule VII
R(4)-L VII
dans laquelle R(4) a la signification donnée dans la revendication 1 à l'exception de l'hydrogène, et L a la signification donnée sous a);
ou **en ce que**
d) dans un composé de formule I 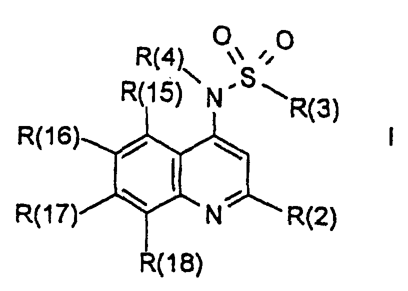
dans laquelle R(2), R(3), R(15), R(16), R(17) et R(18) ont la signification donnée dans la revendication 1, on effectue une réaction de substitution électrophile à au moins l'une des positions R(15), R(16), R(17) et R(18), dans la mesure ou cette position représente un atome d'hydrogène.

3. Composés de formule I selon la revendication 1 et leurs sels physiologiquement acceptables à utiliser comme médicaments.

4. Composition pharmaceutique contenant une quantité active d'au moins un composé de formule I selon la revendication 1 et/ou d'un de ses sels physiologiquement acceptables comme substance active avec des supports et additifs pharmaceutiquement acceptables et éventuellement une ou plusieurs autres substances actives pharmacologiques.

5. Utilisation d'un composé de formule I selon la revendication 1 et/ou d'un de ses sels physiologiquement acceptables pour la préparation d'un médicament à activité de blocage des canaux de K⁺ pour la thérapie et la prophylaxie de maladies ayant pour origine les canaux de K⁺.

6. Utilisation d'un composé de formule I selon la revendication 1 et/ou d'un de ses sels physiologiquement acceptables pour la préparation d'un médicament destiné à l'inhibition de la sécrétion de l'acide gastrique.

7. Utilisation d'un composé de formule 1 selon la revendication 1 et/ou d'un de ses sels physiologiquement acceptables pour la préparation d'un médicament destiné à la thérapie ou à la prophylaxie d'ulcères de l'estomac ou de la région intestinale.

8. Utilisation d'un composé de formule I selon la revendication 1 et/ou d'un de ses sels physiologiquement acceptables pour la préparation d'un médicament destiné à la thérapie ou à la prophylaxie du reflux gastro-oesophagien.

9. Utilisation d'un composé de formule I selon la revendication 1 et/ou d'un de ses sels physiologiquement acceptables pour la préparation d'un médicament destiné à la thérapie ou à la prophylaxie de maladies diarrhéiques.

10. Utilisation d'un composé de formule I selon la revendication 1 et/ou d'un de ses sels physiologiquement acceptables pour la préparation d'un médicament destiné à la thérapie ou à la prophylaxie de tous les types d'arythmies, comprenant les arythmies auriculaires, ventriculaires et supraventriculaires.

11. Utilisation d'un composé de formule I selon la revendication 1 et/ou d'un de ses sels physiologiquement acceptables pour la préparation d'un médicament destiné à la thérapie ou à la prophylaxie des troubles du rythme cardiaque pouvant être éliminés par l'allongement du potentiel d'action.

12. Utilisation d'un composé de formule I selon la revendication 1 et/ou d'un de ses sels physiologiquement acceptables pour la préparation d'un médicament destiné à la thérapie ou à la prophylaxie de la fibrillation auriculaire ou du flutter auriculaire.

13. Utilisation d'un composé de formule I selon la revendication 1 et/ou d'un de ses sels physiologiquement acceptables pour la préparation d'un médicament destiné à la thérapie ou à la prophylaxie des arythmies de ré-entrée ou à l'inhibition de la mort cardiaque soudaine par suite d'une fibrillation ventriculaire.

14. Utilisation d'un composé de formule I selon la revendication 1 et/ou d'un de ses sels physiologiquement acceptables pour la préparation d'un médicament destiné à la thérapie de l'insuffisance cardiaque, en particulier de l'insuffisance cardiaque congestive.

15. Utilisation d'un composé de formule I selon la revendication 1 et/ou d'un de ses sels physiologiquement acceptables pour le blocage du canal de potassium qui est ouvert par l'adénosine-monophosphate cyclique (cAMP).

16. Utilisation d'un composé de formule I selon la revendication 1 et/ou d'un de ses sels physiologiquement acceptables pour la préparation d'un médicament destiné à inhiber la sécrétion stimulée d'acide gastrique, à la thérapie ou à la prophylaxie des ulcères de l'estomac ou de la région intestinale, du reflux gastro-oesophagien, de maladies diarrhéiques, à la thérapie ou à la prophylaxie d'arythmies, comprenant les arythmies auriculaires, ventriculaires et supraventriculaires, de la fibrillation auriculaire et du flutter auriculaire et d'arythmies de ré-entrée, ou à l'inhibition de la mort cardiaque soudaine par suite d'une fibrillation ventriculaire.
